# EUROPÄISCHE PATENTSCHRIFT

(11) **EP 1 265 615 B1**
(45) Veröffentlichungstag und Bekanntmachung des Hinweises auf die Patenterteilung: **19.01.2005**
(21) Anmeldenummer: 01911591.4
(22) Anmeldetag: 05.02.2001
(51) Int. Cl.: A61K 31/55, A61K 9/00

(54) **NEUE KOMBINATION NICHTSEDIERENDER ANTIHISTAMINIKA MIT SUBSTANZEN, DIE DIE LEUKOTRIENWIRKUNG BEEINFLUSSEN, ZUR BEHANDLUNG DER RHINITIS/KONJUNKTIVITIS**
NOVEL COMBINATION OF NON-SEDATIVE ANTI-HISTAMINES CONTAINING SUBSTANCES WHICH INFLUENCE THE ACTION OF LEUKOTRIENE, FOR TREATING RHINITIS/CONJUNCTIVITIS
NOUVELLE COMBINAISON D'ANTIHISTAMINIQUES NON SEDATIFS ET DE SUBSTANCES QUI INFLUENCENT L'EFFET DU LEUCOTRIENE, POUR LE TRAITEMENT DE LA RHINITE/CONJONCTIVITE

(30) Priorität: 17.02.2000 DE 10007203
(43) Veröffentlichungstag der Anmeldung: 18.12.2002
(73) Patentinhaber: VIATRIS GmbH & Co. KG, 60314 Frankfurt am Main (DE)
(72) Erfinder: KUSS, Hildegard, 01109 Dresden (DE); ENGEL, Jürgen, 63755 Alzenau (DE); SZELENYI, Istvan, 90571 Schwaig (DE)
(86) Internationale Anmeldenummer: PCT/EP2001/001190
(87) Internationale Veröffentlichungsnummer: WO 2001/060407

(56) Entgegenhaltungen:
- WO-A-97/28797
- WO-A-98/34611
- WO-A-99/31225
- DR. S.J. TKACHYK: "New treatments for allergic rhinitis" CANADIAN FAMILY PHYSICIAN, Bd. 45, Nr. may, 1999, Seiten 1255-1260, XP001037007
- C. BACHERT ET AL.: "Histamin und Leukotriene bei der allergischen Rhinitis" ALLERGOLOGIE, Bd. 22, Nr. 8, 1999, Seiten 492-507, XP001037614 gent/belgium

## Beschreibung

### Technisches Gebiet

Die vorliegende Erfindung betrifft neue pharmazeutische Zusammensetzungen, die Azelastin sowie eine die Leukotrienwirkung beeinflussende Substanz, die ein Leukotrien D₄-Antagonist, ein 5-Lipoxygenase-Inhibitor, oder ein 5-Lipoxygonase aktivierendes Protein (FLAP)-Antagonist sein kann, enthalten.
Die Kombinationen dienen der Verbesserung der lokalen Therapie der allergischen und/oder vasomotorischen Rhinitis bzw. der allergischen Konjunktivitis. Das Antihistaminikum sorgt für die schnelle Beseitigung der akuten Symptome, die sich als Rötung, Juckreiz oder Schwellung manifestieren, während mit dem in der Kombination enthaltenen Leukotrien-Antagonisten die dem Krankheitsbild zugrunde liegende Entzündung erfolgreich bekämpft wird.

### Stand der Technik

Weltweit nimmt die Anzahl der allergischen Erkrankungen stark zu. Studien haben ergeben, daß weltweit durchschnittlich 7,5 % aller Kinder und Jugendlichen an Rhinokonjunktivitis (Heuschnupfen kombiniert mit einer Augensymptomatik) leiden (Worldwide variation in prevalence of symptoms of asthma, allergic rhinoconjunctivitis and atopic eczema: ISAAC, Lancet, 351, 1225-1332, 1998). In westeuropäischen Ländern ist die Prävalenz mit ca. 14 % deutlich höher (Annesi-Maesano, I. and Oryszczyn, M.P: Rhinitis in adolescents, Results of the ISAAC survey, Revue Francaise d'Allergologie et . d'Immunologie Clinique, 38, 283-289, 1998; Norrman, E., L; Nystrom, E. Jonsson and N. Stjemberg: Prevalence and incidence of asthma and rhinoconjunctivitis in Swedish teenagers, European Journal of Allergy and Clinical Immunology, 53, 28-35, 1998).

Die intensiven Forschungsaktivitäten der letzten Jahre haben zur Erkenntnis geführt, daß es sich bei der allergischen Rhinokonjunktivitis um ein inflammatorisches Geschehen im Sinne einer persistierenden Entzündungsreaktion handelt. Während Histamin nach wie vor als bedeutendster Mediator der Frühphase und als wichtigster Auslöser der Symptome wie Rötung, Niesen, Juckreiz und Hypersekretion (Nasen- bzw. Tränenfluß) angesehen wird, sind weitere Mediatoren wie die Leukotriene an der nasalen Obstruktion, Sekretion und an dem Fortschreiten der Entzündung (z.B. Anlocken der proentzündlichen Zellen, Förderung der zellulären Infiltration, etc.) beteiligt. Dementsprechend haben sich die Ziele der Therapie verlagert, von einer symptomatischen Therapie hin zu einer zusätzlichen antiinflammatorischen Therapie mit Beeinflussung der den allergischen Erkrankungen zugrundeliegenden Entzündung. Sowohl Histamin als auch Leukotriene (LTe) werden in der allergischen Früh- und Spätphase freigesetzt.

Die akuten Symptome (Juckreiz, Rötung, Schwellung, Nasen- bzw, Tränenfluß) der Rhinokonjunktivitis können u.a. mit Hilfe von klassischen Antihistaminika der ersten und der zweiten Generation gut beherrscht werden. Jedoch haben sie kaum einen therapeutisch relevanten Einfluß auf die der Erkrankung zugrunde liegende und stets fortschreitende Entzündung. Oft wird die allergische Rhinitis (Rhinokonjunktivitis) sowohl von Patienten als auch vom Arzt als eine Bagatellerkrankung angesehen und dementsprechend nur unzureichend behandelt. In der Folge kann es jedoch zu einem sog. Etagenwechsel kommen, d.h. aus der relativ harmlosen Rhinitis entwickelt sich ein sehr ernst zu nehmendes Asthma bronchiale. Aus diesem Grunde ist es unerläßlich, bereits die allergische Rhinokonjunktivitis ausreichend und intensiv zu behandeln. Nur dann können die Patienten beschwerdefrei leben und nur dann kann ein u. U. lebensbedrohlicher Etagenwechsel verhindert worden.

Zahlreiche tierexperimentelle und klinische Studien weisen darauf hin, daß sowohl Histamin als auch LTe im Nasensekret nachzuweisen sind (Yamasaki, U., T. Matsumoto, S. Fukuda, T. Natayama, R Nagaya, Y. Ashida. Involvement of thromboxane A2 and histamine in experimental allergic rhinitis of guinea pigs. J. Pharmacol. Exp. Ther. 82:1046, 1997; Pipkom, U, G Karlsson, L Enerbeck. Cellular response of the human allergic nasal mucosa to natural allergen exposure. J. Allergy Clin. Immunol. 35:234, 1988; Volovitz, B., S.L. Osur, M. Berstein, P.L. Ogra. Leukotriene C₄ release in upper respiratory mucosa during natural exposure to ragweed-sensitive children. J. Allergy Clin. Immunol. 82:414, 1988). Durch die Blockierung der Histamin-H₁-Rezeptoren werden bestimmte Symptome wie Niesen, Rötung, Juckreiz und nasale bzw. okuläre Hypersekretion (Nasenfluß, Tränenfluß) signifikant reduziert (Simons, F.E.R., K.J. Simons. Second generation H₁-receptor antagonists. Ann. Allergy 66:5, 1991). In der akuten Phase jeder allergischen Reaktion - unabhängig von der Lokalisation - steht die Degranulation, die Entleerung der intrazellulären Speicher der Mastzellen bzw. der basophilen Granulozyten im Vordergrund. Dabei handelt es sich um einen Vorgang, der durch das extra- bzw. intrazelluläre Kalzium gesteuert wird.

Histamin wirkt jedoch nicht nur als ein Mediator, der allergische Symptome auslöst, es wirkt auch auf die allergische Entzündung, indem es die Freisetzung von Cytokinen beeinflußt. In einer Arbeit an menschlichen konjunktivalen Epithelzellen (Auge) konnte gezeigt werden, daß Histamin die Ausschüttung von IL-8 und GM-CSF ("granulocyte macrophage colony stimulating factor") stark erhöht. Diese Freisetzung kann durch Histamin-H₁-Rezeptorantagonisten verhindert werden, d.h. diese Wirkung wird über H₁-Rezeptoren vermittelt (Weimer, L.K., D.A. Gamache, J.M. Yanni. Histamine-stimulated cytokine secretion from human conjunctival epithelial cells: inhibition by histamine H₁antagonist emedastine. Int. Arch. Allergy Immunol. 115:288, 1998). Weiterhin wissen wir, daß ein allergischer Reiz nicht nur das intrazellulär gespeicherte Histamin aus den Mastzellen und basophilen Granulozyten freisetzt, sondern auch die de novo Synthese von anderen Mediatoren wie Leukotrienen bewirkt.

Leukotriene sind Mediatoren, die zur Gruppe der Eicosanoide gehören. Sie stellen Derivate der Arachidonsäure dar, einer Fettsäure, die Bestandteil von Membranphospholipiden ist. Aus Arachidonsäure werden über die 5-Lipoxygenase (5-LOX) die Leukotriene gebildet. Zum gegenwärtigen Zeitpunkt ist erst die pathogenetisch relevante Rolle der sog. Cysteinyl-Leukotriene abgesichert, zu denen LTC₄, LTD₄ und LTE₄ gehören. Die Wirkung der Leukotriene kann durch Besetzung ihrer Rezeptoren oder durch Hemmung ihrer Synthese erfolgen. Neben der Hemmung der 5-Lipoxygenase kann auch die Hemmung von einem die 5-Lipoxygonase aktivierenden Protein (FLAP) zu einer verminderten Synthese von Leukotrienen führen.
Unter den zahlreichen LT-Antagonisten werden einige wie Zafirtukast, Montelukast, Pranlukast, etc. therapeutisch bei Asthma bronchiale eingesetzt. Von den 5-LOX-Hemmern ist Zileuton bereits auf dem Markt. Zu den sog. FLAP-Inhibitoren gehören z. B. MK-591, Bay x 1005, die sich noch in der klinischen Erprobungsphase befinden.
Zahlreiche Untersuchungen belegen die Bedeutung der Leukotriene bei allergischen Erkrankungen. So konnte nach Allergenprovokation ein deutlicher Anstieg der LT-Konzentration in der nasalen Spülflüssigkeit von Patienten mit allergischer Rhinitis sowohl in der frühen als auch in der späten Phase nachgewiesen worden (Creticos, P.S., S.P. Peters, N.F. Adkinson. Peptide leukotriene release after antigen challenge in patients sensitive to ragweed. N. Eng. J. Med. 310:1626, 1984). Cysteinyl-LTe können eine Hypersekretion (Nasen- bzw. Tränenfluß) auslösen, jedoch weit bedeutender scheinen die Leukotriene für die nasale Obstruktion zu sein.

Die durch Histamin ausgelöste nasale Obstruktion liegt in der Frühphase der allergischen Reaktion und hält nur Minuten an, während die Obstruktion durch Leukotriene bis in die Spätphase, die 6 - 8 Stunden nach der allergischen Provokation zu beobachten ist, anhält. Im Gegensatz zu Histamin treten Niesen und Juckreiz nach LT-Provokation nicht auf (Okuda, M., T. Watase, A. Mazewa, C.M. Liu. The role of leukotrine D₄ in allergic rhinitis. Ann. Allergy 60:537, 1988). Nach Provokation mit LTD₄ kommt es aber zu einer langanhaltenden Einwanderung von eosinophilen Granulozyten, die zum größten Teil für die allergische Entzündung verantwortlich sind (Fujika, M. et al. s. oben). Diese sog. Spätphase-Reaktionen (z.B. nasale Obstruktion) können durch LT-Antagonisten wie z.B. Zafirlukast gebessert werden (Donnelly, A.L., M. Glass, M.C. Minkwitz, T.B. Casale. The leukotriene D₄-receptor antagonist ICI 204219 relieves symptoms of acute seasonal allergic rhinitis. Am. J. Resp. Crit. Care Med. 151:1734, 1995) (ICI. 204219 = Zafirlukast). Auch die 5-LOX-Inhibitoren sind in der Lage, allergische Reaktionen nicht nur im Tierexperiment, sondern auch in der Humantherapie deutlich zu reduzieren (Liu, M.C., L.M, Dube, J. Lancester, and the zileuton study group. Acute and chronic effects of a 5-lipoxygenase inhibitor in asthma: a 6-month randomized multicenter trial. J. Allergy Clin. Immunol. 98:859, 1996).

Azelastin ist derzeit der einzige Wirkstoff unter den Antihistaminika, der sowohl systemisch (Tablette) als auch topisch (Nasenspray und Augentropfen) verfügbar ist. Demzufolge können Patienten auch mit sehr stark ausgeprägten allergischen Symptomen erfolgreich behandelt werden. Dank der verschiedenen pharmazeutischen Formulierungen kann man die Patienten mit Azelastin je nach Art und Schweregrad der Beschwerden individuell behandeln und somit die der Erkrankung zugrundeliegende Entzündung unterdrücken.

Von den modernen Antihistaminika war Azelastin das erste, bei dem die Hemmung der Synthese von den für die allergische Entzündungsreaktion wichtigen Leukotriene in therapeutisch relevanten Dosierungen bzw. Konzentrationen beobachtet wurde (Achterrath-Tuckermann, U., Th. Simmet, W. Luck, I. Szelenyi, B.A. Peskar. Inhibition of cysteinyl-leukotriene production by azelastine and its biological significance. Agents and Actions 24: 217, 1988). Dieser antileukotriene Effekt von Azelastin ist auch in kontrollierten klinischen Studien bei Allergikern nachgewiesen (Shin, M.H., F.M. Baroody, D. Proud, A. Kagey-Sobotka, L.M. Lichtenstein, M. Naclerio. The effect of azelastine on the early allergic response. Clin. Exp. Allergy 22:289, 1992), Durch diese Wirkung läßt sich dann auch die mit Budesonid, einem Glucocorticoid vergleichbare klinische Wirksamkeit von Azelastin erklären (Wang, D.Y., J. Smitz, M, De Waele, P. Clement. Effect of topical applications of budesonide and azelastine on nasal symptoms, easinophil counts and mediator release in atopic patients after nasal allergen challenge during the pollen season. Int. Arch. Allergy Immunol. 114:185, 1997; Gastpar, H., R. Aurich, U. Petzold. Intranasal treatment of perennial rhinitis: Comparison of azelastine nasal spray and budesonide nasal aerosol. Arzn. Forsch. - Drug Res. 43:475, 1993).

Der Wirkungsmechanismus, über den Azelastin die LT-Synthese und die LT-Freisetzung hemmt, ist eigenartig und bei anderen Antihistaminika nicht beschrieben. Bekanntlich verlaufen viele Freisetzungsvorgänge über einen erhöhten Spiegel vom intrazellulären Ca²⁺, der durch eine allergische Stimulation der Effektorzellen zustande kommt, da das intrazelluläre Ca²⁺ die entscheidenden Schritte zur vermehrten Leukotrien-Synthese und -Freisetzung einleitet. Azelastin hemmt die intrazelluläre Ca²⁺-Freisetzung (Takanaka, K. Effects of azelastine on polymorphonuclear leukocytes: arachidonate cascade inhibition mechanism. Progress Med. 275, 1987. Chand, N., *et al.* Inhibition of allergic and nonallergic leukotriene formation and histamine secretion by azelastine: Implication for its mechanism of action. Int. Arch. Allergy Appl. Immunol. 90:67, 1989; Senn, N., *et al.* Action of azelastine on intracellular Ca²⁺ in cultured airway smooth muscle. Eur. J. Pharmacol. 205:29, 1991; Chand, N., R.D. Sofia. A novel in vivo inhibitor of leukotriene biosynthesis: A possible mechanism of action: A mini review. J. Asthm. 32:227, 1995).

Der Wirkungsmechanismus der LT-Rezeptorantagonisten ist "einfach". Als Rezeptorantagonisten besetzen sie die LT-Rezeptoren. Demzufolge können die freigesetzten Leukotriene an ihre Rezeptoren herankommen und ihre durch den Rezeptor zu vermittelnden Wirkungen entfalten.

Kombinationen zur intranasalen Anwendung, die ein Antihistaminikum mit Leukotrien inhibierenden Eigenschaften zusammen mit einem Glukokortikosteroid und gegebenenfalls Dekongestionsmitteln, Antiallergika, Mucolytica, nichtopioiden Analgetika, Lipoxygenase-Inhibitoren und Leukotrien-Rezeptorantagonisten enthalten, sind aus EP 0780127 A1 bekannt und werden zur Behandlung der allergischen Rhinokonjunktivitis empfohlen. Das Zusammenwirken des Antihistaminikums mit dem Glukokortikosteroid soll die Effektivität der Behandlung steigern.

Bekannt ist auch zur topischen Behandlung der Rhinitis aus WO 98148839 die Anwendung eines entzündungshemmenden Mittels in Form von Corticosteroiden, dem zur Steigerung der Wirksamkeit beispielsweise wenigstens ein Vasokonstriktor, ein Leukotrien-Inhibitor, ein Antihistaminikum, ein Antiallergikum, ein Mukolytikum, ein Anästhetikum, ein Anticholinergikum oder ein Neuraminidase-Inhibitor beigegeben werden.

Wie aus WO 98/34611 bekannt ist, werden zur topischen Behandlung des allergischen Asthmas Kombinationen vorgeschlagen, die aus Descarboethoxyloratadin, einem Metaboliten des nichtsedierenden Antihistamins Loratadin und einem Leukotrien-Antagonisten, der ein Leukotrien D₄-Antagonist, ein 5-Lipoxygenase-Inhibitor oder ein FLAP-Antagonist sein kann, bestehen. Die Verwendung des Descarboethoxyloratadins soll eine Vielzahl von unerwünschten Nebenwirkungen des Loratadins sowie anderer nichtsedierender Antistaminika vermeiden.

A. Roquet *et al.,* Combined antagonism of leukotrienes and Histamine produces predominant inhibition of allergen-induced early and late phase airway obstruction in asthmatics. Am. J. Respir. Crit. Care Med., 1997, 155; 1856-1863 untersuchen die Wirkungen des Loratadins, des Leukotrien-Antagonisten Zafirlukast und die Kombination beider Wirkstoffe bei allergeninduzierten Luftwegserkrankungen von Asthmatikern bei oraler Applikation.
Aus Untersuchungen von Merck & Co., WO 97/28797 ist auch bekannt, Loratadin mit fünf ausgewählten Leukotrien-Antagonisten Montelukast, Zafirlukast, Pranlukast, Sodium 1-(((R)-(3-(2-(6,7-difluoro-2-quinolinyl)ethenyl)-phenyl)-3-(2-(2-hydroxy-2-propyl)phenyl)thio)methyl)cyclo-propaneacetate, 1-(((1(R)-(3-(2-(2,3-dichlorothieno[3,2-b]pyridin-5-yl)-(E)-ethenyl)phenyl)-3-(2-(1-hydroxy-1-methylethyl)phenyl)-propyl)thio)methyl)cyclopropaneacetic acid oral oder parenteral bei Asthma, Allergie und Entzündungen anzuwenden.

Zur Behandlung der allergischen Rhinitis/Konjunktivitis besteht aufgrund zahlreicher Nebenwirkungen von eingeführten Präparaten, mangelnder Heilerfolge und der teilweise unspezifischen Therapie weiterhin ein großer Bedarf an Kombinationen mit einer hohen Effektivität und Sicherheit.

Der Erfindung liegt daher die Aufgabe zugrunde, neue Kombinationen zur Behandlung der allergischen Rhinitis/Konjunktivitis zu finden und bereitzustellen.

### Darstellung der Erfindung

Die vorliegende Erfindung betrifft die Bereitstellung von pharmazeutischen Substanzkombinationen, die topisch aber auch oral bei allergischer und/oder vasomotorischer Rhinitis bzw. allergischer Konjunktivitis verabreicht werden können, wobei diese eine wirksame Menge Azelastin in Kombination mit einem die Leukotrienwirkung beeinflussenden Leukotrien D₄-Antagonisten, wie Montelukast, Zafirlukast bzw. Pranlukast oder mit einem 5-Lipoxygenase-Inhibitor, wie Zileuton, Piripost bzw. AWD 23-115 (1-[4-(Chinolin-2-ylmethoxy)-benzyl]-5-methoxy-1H-indazol-3-ol Dihydrochlorid) oder mit einem FLAP-Antagonisten, wie MK-591, MK-886, Bay x 1005 bzw. sowie gegebenenfalls weiterhin pharmazeutisch unbedenkliche Träger und/oder Streckmittel oder Hilfsstoffe hierfür enthalten.

Die vorliegende Erfindung betrifft weiterhin die Bereitstellung geeigneter Einzeldosisformen von Azelastin in Kombination mit einem die Leukotrienwirkung beeinflussenden Leukotrien D₄-Antagonisten, wie Montelukast, Zafirlukast bzw. Prantukast oder mit einem 5-Lipoxygenase-Inhibitor, wie Zileuton, Piripost bzw. AWD 23-115 oder mit einem FLAP-Antagonisten, wie MK-591, MK-886, Bay x 1005, die sich für eine bequeme topische oder orale Verabreichung eignen, zum Beispiel in Form von Sprays oder Tropfen oder Tabletten.

Die neue Kombination aus Azelastin und einem die Leukotrienwirkung beeinflussenden Leukotrien D₄-Antagonisten, wie Montelukast, Zafirlukast bzw. Pranlukast oder einem 5-Lipoxygenase-Inhibitor, wie Zileuton, Piripost bzw. AWD 23-115 oder einem FLAP-Antagonisten, wie MK-591, MK-886 bzw. Bay x 1005, die auch als pharmazeutisch unbedenkliche Salze vorliegen können, kann erfindungsgemäß als fixe Kombination oder in Einzelsubstanzen gleichzeitig, nacheinander oder unabhängig voneinander topisch (intranasal oder intraoculär) oder oral gegeben werden.
Liegen getrennte Formulierungen vor, dann sind diese aufeinander abgestimmt und enthalten die jeweiligen Wirkstoffe in der Dosierungseinheit in den gleichen Mengen und entsprechenden Gewichtsverhältnissen, in denen sie in der Kombination enthalten sein können.
Durch die Kombination kommt es nicht nur zu einem schnellen Wirkungseintritt, sondern auch zu einer hohen therapeutischen Wirksamkeit, die mit einer starken antientzündlichen Wirkung einher geht, da sich die Wirkungsweisen der genannten Wirkstoffe gegenseitig ergänzen und sich auch pharmakokinetisch ähnlich verhalten. Die lange Wirkdauer ermöglicht eine zweimal tägliche Gabe. Wenn die wirksamen Komponenten in Form einer fixen Kombination vorliegen, ist die Anwendung für den Patienten einfacher, denn beide Wirkstoffe sind in einer Tablette bzw. einem Behälter enthalten.
Die Konzentration von Azelastin kann im Bereich von 0,001 % bis 0,5 % liegen.
Die Konzentration der Leukotrien-Antagonisten in der Kombination kann im Bereich von 0,01 % bis 5 % liegen.
Bevorzugte Konzentrationen betragen 0,05 % bis 0,2 % für Azelastin und 0,5 % bis 2 % für Leukotrien-Antagonisten.

Die vorgesehene Dosierung erfolgt ein- bis zweimal täglich. Die Einzeldosis von Azelastin beträgt 50 - 500 µg, bevorzugt 200 - 400 µg topisch appliziert. Die Dosis vom Leukotrien D₄-Antagonisten liegt bei topischer Applikation zwischen 100 - 2000 µg, bevorzugt 200 - 1000 µg. 5-LOX- bzw. FLAP-Inhibitoren werden in einem Dosisbereich von 50 - 2000 µg, bevorzugt 200 - 1000 µg appliziert.

Die Dosis von Azelastin liegt zwischen 0,5 -16 mg/Tag, vorzugsweise 2 - 8 mg/Tag. Bei den Leukotrien D₄-Antagonisten (zum Beispiel Montelukast) beträgt die Einzeldosis 1 - 50 mg/Tag, bevorzugt 5 - 10 mg/Tag.
Die orale Dosis von 5-LOX-tnhibitoren wie zum Beispiel Zileuton liegt zwischen 1 - 6 g/Tag, bevorzugt 0,6 - 2 g/Tag.
Bei FLAP-Inhibitoren beträgt die Dosis 50 - 2000 mg/Tag, vorzugsweise 100 - 500 mg/Tag.

Die genannten Verbindungen des Azelastin und der Leukotrien-Antagonisten und Verfahren zu ihrer Herstellung sind bekannt.

Die pharmazeutische und galenische Handhabung der Verbindungen zu Kombinationen erfolgt nach den üblichen Standardmethoden, indem vorzugsweise Azelastin und der Leukotrien-Antagonist einzeln oder zusammen, gegebenenfalls zusammen mit Trägern und/oder Streckmitteln oder Hilfsstoffen vermischt und die so erhaltene Mischung in geeignete Darreichungsformen überführt wird.
Die Wirkstoffe werden in Form einer Mischung, die für pharmazeutische Zwecke übliche pharmazeutische Streckmittel, Excipienten oder Träger enthält, oral oder topisch verabreicht.

Die Zusammensetzungen zur oralen oder topischen Verabreichung können als unterschiedliche, pharmazeutisch unbedenkliche Darreichungsformen, z. B.
Nasensprays, Nasentropfen, Augentropfen, Tabletten, Kapseln oder Granulate formuliert werden.
Außer den Wirkstoffen können die erfindungsgemäßen Zusammensetzungen weiterhin verschiedene arzneiformtypische Bestandteile wie antimikrobielle Konservierungsmittel, Osmotika, Verdickungsmittel, Excipienten für die pH-Einstellung oder Puffersysteme enthalten.

Zu den antimikrobiellen Konservierungsstoffen zählen zum Beispiel Benzalkoniumchlorid, Cetylpyridiniumchlorid/bromid, Chlorbutanol, Chlorhexidinacetat, Chlorhexidin-HCl, Chlorhexidindigluconat, Chlorkresol, Methylparaben, Propylparaben, Phenoxyethanol, Phenylquecksilbersalze, Sorbinsäure, Thiomersal.

Für Konservierungszwecke wird vorzugsweise eine Kombination von Natriumedetat und Benzalkoniumchlorid verwendet. Natriumedetat wird in Konzentrationen von 0,05 - 0,1 % und Benzalkoniumchlorid in Konzentrationen von 0,005 - 0,05 % verwendet.

Zu den geeigneten Excipienten, die sich zur Einstellung der Tonizität oder Osmolalität eignen, können Natriumchlorid, Kaliumchlorid, Mannit Glucose, Sorbit, Glycerin oder Propylenglykol in Konzentrationen von etwa 0,1 bis 10 % verwendet werden.

Häufig enthalten die Zusammensetzungen Verdickungsmittel, um die Viskosität zu erhöhen und den Kontakt zwischen Arzneistoff und Körpergewebe zu verlängern und zu verbessern.
Zu diesen Verdickungsmitteln zählen Methylcellulose, Hydroxypropylmethylcellulose, Hydroxyethylcellulose, Natriumcarboxymethylcellulose, Polyvinylalkohol, Polyvinylpyrrolidon, Polyacrylate, Polyacrylamid, Dextran, Gellangummi, Poloxamer oder Celluloseacetatphthalat.
Außerdem beinhalten die erfindungsgemäßen Zusammensetzungen pharmazeutisch unbedenkliche Puffer, daß der pH auf einen Bereich von ungefähr 4 bis 8, vorzugsweise 5,5 bis 7,5 eingestellt und gehalten werden kann. Solche Puffer sind Zitrat, Phosphat, Tromethamin, Glycin, Borat oder Acetat.
Diese Puffer können sich auch von solchen Substanzen wie Zitronensäure, primärem oder sekundärem Natriumphosphat, Glycin, Borsäure, Natriumtetraborat, Essigsäure und Natriumacetat ableiten. Außerdem können weitere Excipienten wie Salzsäure oder Natriumhydroxid der pH-Einstellung dienen.

Die vorliegende Erfindung soll anhand einiger Beispiele erläutert werden.

### Beispiel 1:

Nasenspray bzw. Nasentropfen enthaltend Azelastinhydrochlorid (0,1 %)

| | |
|---|---|
| Azelastinhydrochlorid | 0,1000 g |
| Hydroxxypropylmethylcellulose | 0,1000 g |
| Natriumedetat | 0,0500 g |
| Benzalkoniumchlorid | 0,0125 g |
| Natriumhydroxid | q. s. ph 6,0 |
| Sorbitol-Lösung 70 % | 6,6666 g |
| Gereinigtes Wasser | ad 100 ml |

In einem geeigneten Rührwerksbehälter ca. 45 kg gereinigtes Wasser vorlegen. Darin den Wirkstoff, Hydroxypropylmethylcellulose, Natriumedetat, Benzalkoniumchlorid und Sorbitol-Lösung nacheinander zugeben und unter Rühren auflösen. Die entstandene Lösung mit gereinigtem Wasser auf ein Volumen von 49,5 Liter auffüllen. Den pH-Wert der Lösung mit 1 N Natronlauge auf pH 6,0 einstellen. Mit gereinigtem Wasser auf das Endvolumen von 50,0 Liter auffüllen und rühren. Die Lösung durch ein Membranfilter mit einer Porengröße von 0,2 µm filtrieren und in Flaschen abfüllen.

### Beispiel 2:

Nasenspray- bzw. Nasentropfen-Suspension mit Montelukast (1 %)

| | |
|---|---|
| Montelukast | 1,0000 g |
| Avicel RC 591 | 1,1000 g |
| Polysorbat 80 | 0,1000 g |
| Sorbitol-Lösung 70 % | 6,0000 g |
| Natriumedetat | 0,0500 g |
| Benzalkoniumchlorid | 0,0200 g |
| Gereinigtes Wasser | ad 100 ml |

### Herstellung:

In einem geeigneten Rührwerksbehälter mit Homogenisiereinrichtung 45 kg gereinigtes Wasser vorlegen und darin Avicel RC 591 hochtourig einhomogenisieren. Danach nacheinander die Stoffe Polysorbat 80, Sorbitol-Lösung, Natriumedetat und Benzalkoniumchlorid unter Rühren auflösen. Anschließend den Wirkstoff Montelukast hochtourig einhomogenisieren, bis eine gleichmäßige Suspension entstanden ist. Danach auf das Endvolumen von 50 Liter mit gereinigtem Wasser auffüllen und weiter homogenisieren. Anschließend die Suspension evakuieren, um die entstandenen Luftblasen zu entfernen. Die entstandene Suspension wird anschließend in Flaschen abgefüllt.

### Beispiel 3:

Nasenspray bzw. Nasentropfen enthaltend Azelastinhydrochlorid (0,1 %, gelöst) und Montelukast (1 %. suspendiert)

| | |
|---|---|
| Montelukast | 1,0000 g |
| Azelastinhydrochlorid | 0,1000 g |
| Avicel RC 591 | 1,1000 g |
| Polysorbat 80 | 0,1000 g |
| Sorbitol-Lösung 70 % | 6,0000 g |
| Natriumedetat | 0,0500 g |
| Benzalkoniumchlorid | 0,0200 g |
| gereinigtes Wasser | ad 100 ml |

### Herstellung:

In einem geeigneten Rührwerksbehälter mit Homogenisierungseinrichtung 45 kg gereinigtes Wasser vorlegen und darin Avicel RC 591 hochtourig einhomogenisieren. Danach nacheinander den Wirkstoff Azelastinhydrochlorid sowie die Hilfsstoffe Polysorbat 80, Sorbitol-Lösung, Natriumedetat und Benzalkoniumchlorid unter Rühren auflösen. Anschließend den Wirkstoff Montelukast hochtourig einhomogenisieren, bis eine gleichmäßige Suspension entstanden ist. Danach auf das Endvolumen von 50 Liter mit gereinigtem Wasser auffüllen und weiter homogenisieren. Anschließend die Suspension evakuieren, um die entstandenen Luftblasen zu entfernen. Die entstandene Suspension wird anschließend in Flaschen abgefüllt.

Aus den Wirkungsspektren einiger Antihistaminika und auch LT-Antagonisten bzw. 5-LOX- und FLAP-Inhibitoren läßt sich ableiten, daß eine Kombination beider Substanzen eine synergistische Wirkung auf die Symptome der allergischen Rhinokonjunktivitis entfaltet.

Die folgende pharmakologische Untersuchung beschreibt die Wirkung von Azelastin und Montelukast allein und in ihrer Kombination an einem Rhinitis-Modell an Brown Norway Ratten. Die Brown Norway Ratten wurden durch zweimalige i.p. Injektion einer Suspension aus Ovalbumin und Aluminiumhydroxid in physiologischer Kochsalzlösung an zwei aufeinanderfolgenden Tagen aktiv sensibilisiert. Drei Wochen nach der Sensibilisierung wurde den Tieren in Natriumthiopental-Narkose ein Katheter orthograd in die Trachea eingebunden, um die Atmung der Tiere aufrecht zu erhalten und ein weiterer Katheter zur Perfusion der Nasenhöhlen retrograd über die Trachea bis zur inneren Öffnung der Choanen vorgeschoben und fixiert. Das nasale Perfusat kann so über die Nasenlöcher austropfen und über einen Fraktionssammler aufgenommen werden. Die Testsubstanzen wurden entweder in Tylose aufgeschlemmt (Montelukast) oder in physiologischer Kochsalzlösung gelöst (Azelastin) und 60 min vor der Allergen-Provokation intraperitoneal injiziert. Um Schleim aus der Nase wegzuspülen, wurde PBS über 30 min mit einer Rollenpumpe durch die Nasenhöhle perfundiert (Perfusionsgeschwindigkeit 0,5 ml/min). Bei topischer Applikation werden die Testsubstanzen in molarer Konzentration dem Perfusat zugesetzt und über 30 min vor der Allergen-Provokation die Lösung durch die Nase perfundiert. Anschließend wurde der Plasmamarker Evans blue (je 1 ml/Tier einer 1 %igen Lösung in PBS) in die Vena jugularis injiziert. Danach erfolgte eine Pause von 15 min, in der die Perfusionsflüssigkeit gesammelt wurde. Die Allergen-Provokation (Challenge) erfolgte anschließend durch Perfusion der Nasenhöhle mit einer Lösung aus Ovalbumin in PBS (10 mg/ml Ovalbumin in PBS) über 60 min, bei der das Perfusat in 15 min Fraktionen in dem Fraktionssammler gesammelt wurde. Die Gesamtmenge der Proben/Tier betrug 5. Die Proben wurden zentrifugiert und anschließend auf Mikrotiterplatten aufgetragen und mit dem Photometer Digiscan bei einer Wellenlänge von 620 nm gemessen. Dabei wurden die Blankwerte automatisch abgezogen. Der Wirkungsverlauf über 60 min wurde mit einem AUC-Programm berechnet. Die Substanzwirkung der Präparategruppe wurde gegen Vehikelkontrollen in % berechnet.

Eine erhöhte mukosale Durchlässigkeit nach Allergen-Provokation ist als Zeichen der Freisetzung von Botenstoffen wie Histamin und Leukotriene zu werten. Dieses Phänomen kommt nach Antigen-Kontakt auch bei Allergikern vor und äußert sich in vermehrter Flüssigkeitssekretion und nasaler Verstopfung.

**Tabelle 1:**

| Wirkung von Azelastin und Montelukast allein und in Kombination (intraperitoneale Applikation) auf die nasale mukosale Permeabilität in aktiv sensibilisierten und topisch provozierten Brown-Norway Ratten | | |
|---|---|---|
| Substanz | Dosis (mg/kg, i.p.) | Hemmwirkung in % |
| Azelastin | 0,01 | 11 |
| | 0,1 | 39 |
| | 0,3 | 42 |
| | 1 | 47 |
| | | |
| Montelukast | 0,1 | 7 |
| | 1 | 26 |
| | 3 | 39 |
| | 10 | 44 |
| | 30 | 58 |
| | | |
| Azelastin + Montelukast | 0,01 + 0,1 | 40* |

| | | |
|---|---|---|
| *p<0.05 | | |

Die alleinige Applikation von Azelastin bewirkt in der Dosis 0,01 mg/kg i.p. eine geringe Hemmung der vaskulären Permeabilität von 11%. Montelukast ist in der Dosis 0,1 mg/kg i.p. mit 7% Hemmung ebenfalls schwach wirksam. Die kombinatorische Gabe von Azelastin in der Dosis 0,01 mg/kg i.p. und Montelukast in der Dosis 0,1 mg/kg i.p. bewirkte eine überadditive Hemmung der mukosalen Plasmaextravasation von 40% (p<0,05).

Der FLAP-Inhibitor BAY x 1005 hemmte die nasale mukosale Permeabilität in der Dosis 0,1 mg/kg i.p. um 31%. AWD 23-115, ein 5-LOX-Inhibitor bewirkte im Dosisbereich von 0,03 bis 10 mg/kg i.p. eine dosisabhängige Hemmung (37 - 54%) der vaskulären Permeabilität.

**Tabelle 2:**

| Wirkung von Azelastin und AWD 23-115 allein und in Kombination (topische Applikation im Perfusat) auf die nasale mukosale Permeabilität in aktiv sensibilisierten und topisch provozierten Brown-Norway Ratten | | |
|---|---|---|
| Substanz | Konzentration (µmol/l) | Hemmwirkung in % |
| Azelastin | 0,003 | 3 |
| | 0,01 | 40 |
| | 0,03 | 60 |
| | | |
| AWD 23-115 | 0,1 | 12 |
| | 0,3 | 32 |
| | 1 | 49 |
| | | |
| Azelastin + AWD 23-115 | 0,003 + 0,1 | 31* |
| p < 0,05 | | |

Der Histamin H₁-Blocker Azelastin zeigt bei topischer Applikation bereits in Konzentrationen von 0,003 bis 0,03 µmol/l eine starke Hemmung der mukosalen Plasmaextravasation. Der 5-LOX-Hemmer AWD 23-115 hemmt die. vaskuläre Permeabilität bei 0,3 und 1 µmol/l dosisabhängig um 32% bzw. 49%. Wenn Azelastin bei einer Konzentration von 0,003 µmol/l in Kombination mit AWD 23-115 (0,1 µmol/l) gegeben wird, beträgt die Hemmung der mukosalen Extravasation 31% (p<0,05).

## Patentansprüche

1. Pharmazeutische Zubereitung zur Behandlung von allergischer und/oder vasomotorischer Rhinitis bzw. allergischer Konjunktivitis, die sich zur topischen oder oralen Verabreichung von Einzeldosen eignet, mit folgenden Bestandteilen, getrennt oder zusammen:
a) eine wirksame Menge Azelastin.
b) eine wirksame Menge eines Leukotrien-Antagonisten oder eines seiner pharmazeutisch unbedenklichen Salze, ausgewählt aus einer Gruppe, die besteht aus
b1) einem Leukotrien D₄-Antagonisten oder
b2) einem 5-Lipoxygenase-Inhibitor oder
b3) einem FLAP-Antagonisten
c) übliche physiologisch unbedenkliche Träger und/oder Streckmittel oder Hilfsstoffe.

2. Pharmazeutische Zubereitung nach Anspruch 1, wobei der Leukotrien D₄-Antagonist Montelukast, Zafirlukast oder Pranlukast umfaßt.

3. Pharmazeutische Zubereitung nach Anspruch 1, wobei der 5-Lipoxygenase- Inhibitor Zileuton, Piripost oder AWD 23-115 umfaßt.

4. Pharmazeutische Zubereitung nach Anspruch 1, wobei der FLAP-Antagonist MK-591, MK-886 oder Bay x 1005 umfaßt.

5. Pharmazeutische Zubereitung nach den Ansprüchen 1 und 2 mit 0,001 % bis 0, 5% Azelastin.

6. Pharmazeutische Zubereitung nach den Ansprüchen 1 und 3 mit 0,01 % bis 5 % der Komponente Leukotrien D₄-Antagonist.

7. Pharmazeutische Zubereitung nach den Ansprüchen 1 und 4 mit 0,01 % bis 5 % der Komponente 5-Lipoxygenase-Inhibitor.

8. Pharmazeutische Zubereitung nach den Ansprüchen 1 und 5 mit 0,01 % bis 5 % der Komponente FLAP-Antagonist.

9. Pharmazeutische Zubereitung nach den Ansprüchen 1 bis 8 in topischer Darreichungsform.

10. Pharmazeutische Zubereitung nach den Ansprüchen 1 bis 8 in oraler Darreichungsform.

11. Pharmazeutische Zubereitung nach den Ansprüchen 1 bis 9 in der topischen Darreichungsform eines Sprays.

12. Pharmazeutische Zubereitung nach den Ansprüchen 1 bis 9 in der topischen Darreichungsform von Nasen- oder Augentropfen.

13. Arzneimittel zur topischen oder oralen Verabreichung mit einer Wirkung gegen allergische und/oder vasomotorische Rhinitis bzw. allergische Konjunktivitis mit folgenden Bestandteilen in fixer oder freier Kombination:
a) eine wirksame Menge Azelastin.
b) eine wirksame Menge eines Leukotrien-Antagortisten oder eines seiner pharmazeutisch unbedenklichen Salze, ausgewählt aus einer Gruppe, die besteht aus
b1) einem Leukotrien D₄-Antagonisten oder
b2) einem 5-Lipoxygenase-Inhibitor oder
b3) einem FLAP-Antagonisten, sowie
c) gegebenenfalls übliche physiologisch unbedenkliche Träger und/oder Streckmittel oder Hilfsstoffe.

14. Arzneimittel nach Anspruch 13, wobei der
- Leukotrien D₄-Antagonist Zafirlukast, Montelukast oder Pranlukast,
- 5-Lipoxygenase-Inhibitor Zileuton, Piripost oder AWD 23-115,
- FLAP-Antagonist MK-591, MK-886 oder Bay x 1005 umfaßt.

15. Verfahren zur Herstellung eines Arzneimittels gemäß den Ansprüchen 13 oder 14, **dadurch gekennzeichnet, daß** das Antihistaminikum und der Leukotrien-Antagonist einzeln oder zusammen, gegebenenfalls zusammen mit Trägem und/oder Streckmitteln oder Hilfsstoffen verarbeitet und die so erhaltene Mischung in geeignete Darreichungsformen überführt wird.

## Claims

1. Pharmaceutical preparation for the treatment of allergic and/or vasomotor rhinitis or allergic conjunctivitis which is suitable for topical or oral administration of single doses, with the following ingredients, separately or together:
a) an effective amount of azelastine,
b) an effective amount of a leukotriene antagonist or of one of its pharmaceutically acceptable salts selected from a group consisting of
b1) a leukotriene D₄ antagonist or
b2) a 5-lipoxygenase inhibitor or
b3) a FLAP antagonist
c) conventional physiologically acceptable carriers and/or extenders or excipients.

2. Pharmaceutical preparation according to Claim 1, where the leukotriene D₄ antagonist comprises montelukast, zafirlukast or pranlukast.

3. Pharmaceutical preparation according to Claim 1, where the 5-lipoxygenase inhibitor comprises zileuton, piripost or AWD 23-115.

4. Pharmaceutical preparation according to Claim 1, where the FLAP antagonist comprises MK-591, MK-886 or Bay x 1005.

5. Pharmaceutical preparation according to Claims 1 and 2 with 0.001% to 0.5% azelastine.

6. Pharmaceutical preparation according to Claims 1 and 3 with 0.01% to 5% of the leukotriene D₄ antagonist component.

7. Pharmaceutical preparation according to Claims 1 and 4 with 0.01% to 5% of the 5-lipoxygenase inhibitor component.

8. Pharmaceutical preparation according to Claims 1 and 5 with 0.01% to 5% of the FLAP antagonist component.

9. Pharmaceutical preparation according to Claims 1 to 8 in topical dosage form.

10. Pharmaceutical preparation according to Claims 1 to 8 in oral dosage form.

11. Pharmaceutical preparation according to Claims 1 to 9 in the topical dosage form of a spray.

12. Pharmaceutical preparation according to Claims 1 to 9 in the topical dosage form of nose or eye drops.

13. Medicament for topical or oral administration having an effect on allergic and/or vasomotor rhinitis or allergic conjunctivitis with the following ingredients in fixed or free combination:
a) an effective amount of azelastine,
b) an effective amount of a leukotriene antagonist or of one of its pharmaceutically acceptable salts selected from a group consisting of
b1) a leukotriene D₄ antagonist or
b2) a 5-lipoxygenase inhibitor or
b3) a FLAP antagonist and
c) where appropriate conventional physiologically acceptable carriers and/or extenders or excipients.

14. Medicament according to Claim 13, where the
- leukotriene D₄ antagonist comprises zafirlukast, montelukast or pranlukast,
- 5-lipoxygenase inhibitor comprises zileuton, piripost or AWD 23-115,
- FLAP antagonist comprises MK-591, MK-886 or Bay x 1005.

15. Process for producing a medicament according to Claims 13 or 14, **characterized in that** the antihistamine and the leukotriene antagonist is processed singly or together, where appropriate together with carriers and/or extenders or excipients, and the mixture obtained in this way is converted into suitable dosage forms.

## Revendications

1. Préparation pharmaceutique destinée au traitement de la rhinite allergique et/ou vasomotrice ou de la conjonctivite allergique, qui convient à l'administration topique ou orale de doses unitaires, comportant les composants suivants, séparés ou ensemble :
a) une quantité efficace d'azélastine,
b) une quantité efficace d'un antagoniste de leucotriène ou d'un de ses sels pharmaceutiquement acceptables, choisi dans un groupe qui est constitué par
b1) un antagoniste de leucotriène D₄,
b2) un inhibiteur de 5-lipoxygénase et
b3) un antagoniste de FLAP (protéine activatrice de 5-lipoxygénase),
c) des véhicules et/ou diluants ou adjuvants, physiologiquement acceptables.

2. Préparation pharmaceutique selon la revendication 1, dans laquelle l'antagoniste de leucotriène D₄ comprend le montélukast, le zafirlukast ou le pranlukast.

3. Préparation pharmaceutique selon la revendication 1, dans laquelle l'inhibiteur de 5-lipoxygénase est le zileuton, le piripost ou AWD 23-115.

4. Préparation pharmaceutique selon la revendication 1, dans laquelle l'antagoniste de FLAP comprend MK-591, MK-886 et Bay x 1005.

5. Préparation pharmaceutique selon les revendications 1 et 2, comprenant de 0,001 % à 0,5 % d'azélastine.

6. Préparation pharmaceutique selon les revendications 1 et 3, comprenant de 0,01 % à 5 % du composant antagoniste de leucotriène D₄.

7. Préparation pharmaceutique selon les revendications 1 et 4, comprenant de 0,01 % à 5 % du composant inhibiteur de 5-lipoxygénase.

8. Préparation pharmaceutique selon les revendications 1 et 5, comprenant de 0,01 % à 5 % du composant antagoniste de FLAP.

9. Préparation pharmaceutique selon les revendications 1 à 8, sous forme d'administration topique.

10. Préparation pharmaceutique selon les revendications 1 à 8, sous forme d'administration orale.

11. Préparation pharmaceutique selon les revendications 1 à 9, sous la forme d'administration topique d'une composition à pulvériser en aérosol.

12. Préparation pharmaceutique selon les revendications 1 à 9, sous la forme d'administration topique de gouttes pour le nez ou de collyres.

13. Médicament pour administration topique ou orale, ayant une action contre la rhinite allergique et/ou vasomotrice ou la conjonctivite allergique, comportant les composants suivants, en association fixe ou libre :
a) une quantité efficace d'azélastine,
b) une quantité efficace d'un antagoniste de leucotriène ou d'un de ses sels pharmaceutiquement acceptables, choisi dans un groupe qui est constitué par
b1) un antagoniste de leucotriène D₄,
b2) un inhibiteur de 5-lipoxygénase et
b3) un antagoniste de FLAP, ainsi que
c) éventuellement des véhicules et/ou diluants ou adjuvants usuels, physiologiquement acceptables.

14. Médicament selon la revendication 13, dans lequel
- l'antagoniste de leucotriène D₄ comprend le zafirlukast, le montélukast ou le pranlukast,
- l'inhibiteur de 5-lipoxygénase comprend le zileuton, le piripost ou AWD 23-115,
- l'antagoniste de FLAP comprend MK-591, MK-886 et Bay x 1005.

15. Procédé pour la fabrication d'un médicament selon la revendications 13 ou 14, **caractérisé en ce que** l'antihistaminique et l'antagoniste de leucotriène sont mis en oeuvre individuellement ou ensemble, éventuellement conjointement avec des véhicules et/ou diluants ou adjuvants, et le mélange ainsi obtenu est transformé en des formes d'administration appropriées.
